(19) Europäisches Patentamt
European Patent Office
Office européen des brevets

(11) Veröffentlichungsnummer: **0 174 574**
**A2**

(12) # EUROPÄISCHE PATENTANMELDUNG

(21) Anmeldenummer: 85110992.6

(22) Anmeldetag: 31.08.85

(51) Int. Cl.[4]: A 01 N 43/66
C 07 D 251/46

(30) Priorität: 13.09.84 DE 3433546

(43) Veröffentlichungstag der Anmeldung: 19.03.86 Patentblatt 86/12

(84) Benannte Vertragsstaaten: AT BE CH DE FR GB IT LI NL

(71) Anmelder: BAYER AG
Konzernverwaltung RP Patentabteilung
D-5090 Leverkusen 1 Bayerwerk(DE)

(72) Erfinder: Schwamborn, Michael, Dr.
von-Lohe-Strasse 9
D-5000 Köln 80(DE)

(72) Erfinder: Kühle, Engelbert, Dr.
von-Bodelschwingh-Strasse 42
D-5060 Bergisch-Gladbach 2(DE)

(72) Erfinder: Brandes, Wilhelm, Dr.
Eichendorffstrasse 3
D-5653 Leichlingen 1(DE)

(72) Erfinder: Köller, Wolfram, Dr.
Hohenzollernring 94
D-5000 Köln 1(DE)

(54) **Fungizide Mittel auf Triazin-Derivat-Basis.**

(57) Die Erfindung betrifft die Verwendung teilweise bekannter Triazin-Derivate der Formel (I)

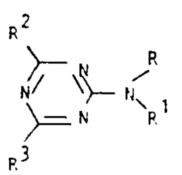

(I)

in welcher

R für Wasserstoff, Alkyl oder Alkenyl steht,

$R^1$ für Wasserstoff, Alkyl, Alkenyl, Alkinyl, Alkoxyalkyl, Alkoxycarbonyl, Alkoxycarbonylalkyl, Carboxyalkyl, Aralkyl oder gegebenenfalls ein- bis mehrfach, gleich oder verschieden substituiertes Cycloalkyl steht oder

R und $R^1$ gemeinsam mit dem Stickstoffatom, an dem sie stehen, einen gesättigten Heterocyclus bilden, der weitere Heteroatome enthalten kann und

$R^2$ und $R^3$ gleich oder verschieden sind und für Alkoxy, Alkylthio oder Phenylthio stehen,

als Fungizide, vor allem mit einer Wirkung gegen BCM-resistente Pilzstämme.

EP 0 174 574 A2

BAYER AKTIENGESELLSCHAFT 5090 Leverkusen, Bayerwerk
Konzernverwaltung RP
Patentabteilung Bas/Em-c
II

## Fungizide Mittel auf Triazin-Derivat-Basis

Die vorliegende Erfindung betrifft die Verwendung von teilweise bekannten Triazin-Derivaten als Fungizide.

Triazin-Derivate, wie das 2-iso-Propylamino-4,6-bismethylthio-1,3,5-triazin, sind bekannt (vgl. K. Stammbach u. a. "Weed Res. 4, 64-74). Die genannte Verbindung tritt als Verunreinigung auf. Das 2-Cyclohexylamino-4,6-bis-methylthio-1,3,5-triazin ist aus der Polymerchemie bekannt (vgl. DE-OS 2.309.105).

Ferner sind 2-Alkoxycarbonylamino-1,3,5-triazine bekannt. Sie zeigen antiinflammatorische, analgetische und sedative Eigenschaften (vgl. DE-OS 2 453 142).

Weiterhin ist bekannt, daß Benzimidazolcarbamate, wie z. B. das Methylbenzimidazol-2-ylcarbamat (allgemein als BCM bekannt), und Phenylendiaminthioharnstoffcarbamate, wie z.B. 1,2-Bis-(3-ethoxycarbonyl-2-thioureido)-benzol, eine ausgezeichnete Wirkung gegen die verschiedensten pflanzenpathogenen Pilze zeigen. Sie werden seit über einem Jahrzehnt als Pflanzenfungizide eingesetzt. Allerdings hat der Einsatz dieser hochaktiven Fungizide über einen längeren Zeitraum Probleme mit sich gebracht,

es wurde Resistenz bei den verschiedensten Mikroorganismen beobachtet.

Weiterhin wurde zwischen Benzimidazolcarbamaten und Phenylendiaminthioharnstoffcarbamaten eine weitgehende Crossresistenz nachgewiesen (vgl. z.B. M. C. Brown u. a. "Plant. Pathol., 33 (1), 101-111 = C.A. 100 (23): 187.178n; C.A. 100 (7): 46.896h und C.A. 100 (1) : 2.204 b).

Weiterhin ist bekannt, daß bestimmte N-Phenylcarbamate, wie z.B. Ethyl-N-(3,5-diisopropyloxyphenyl)-carbamat, eine fungizide Wirkung gegen Benzimidazolcarbamat-Derivatresistente Pilzstämme besitzen (vgl. EP-OS 0 051 871 und EP-OS 0 063 905).
Die erfindungsgemäß anzuwendenden Stoffe zeigen jedoch den Vorteil, daß sie eine ausgezeichnete fungizide Wirkung vor allem gegen BCM-resistente Pilzstämme aufweisen.
Es wurde gefunden, daß die teilweise bekannten Triazin-Derivate der Formel (I)

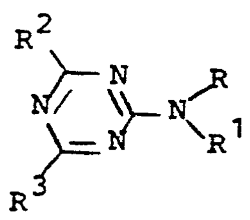

(I)

in welcher

R für Wasserstoff, Alkyl oder Alkenyl steht,

$R^1$ für Wasserstoff, Alkyl, Alkenyl, Alkinyl, Alkoxyalkyl, Alkoxycarbonyl, Alkoxycarbonylalkyl, Carboxyalkyl, Aralkyl oder gegebenenfalls ein- bis mehrfach, gleich oder verschieden substituiertes Cycloalkyl steht, oder

R und $R^1$ gemeinsam mit dem Stickstoffatom, an dem sie stehen, einen gesättigten Heterocyclus bilden, der weitere Heteroatome enthalten kann und

$R^2$ und $R^3$ gleich oder verschieden sind und für Alkoxy, Alkylthio oder Phenylthio stehen,

eine fungizide Wirkung insbesondere gegen BCM-resistente Pilzstämme aufweisen.

Überraschenderweise zeigen die erfindungsgemäß verwendeten Triazin-Derivate eine ausgezeichnete, spezifische Wirkung gegen pilzliche Isolate mit Resistenz gegen BCM.

Unter BCM-resistenten Pilzstämmen verstehen wir in diesem Zusammenhang Pilzstämme, die gegen BCM, BCM-Analoge und gegen Verbindungen, die vor, während oder nach der Applikation BCM bilden, wie z.B. Phenylendiaminthioharnstoffcarbamate, resistent sind.

Die erfindungsgemäß zu verwendenden Triazin-Derivate sind durch die Formel (I) genau definiert und sind teilweise bekannt (vgl. DE-OS 2.309.105; H.Hlawatschek u.a., "Zeitschrift Anorg. Allg. Chem. 504, 201; Nippon Dojo-Hiryogaku Zasshi 41, 4, 133-141; K. Stammbach u.a. "Weed. Res. 4, 64-67" oder DE-OS 2 453 142).

In der Formel (I) stehen vorzugsweise

R für Wasserstoff, für geradkettiges oder verzweigtes Alkyl mit 1 bis 8 Kohlenstoffatomen oder für geradkettiges oder verzweigtes Alkenyl mit 3 bis 6 Kohlenstoffatomen,

$R^1$ für Wasserstoff, für geradkettiges oder verzweigtes Alkyl mit 1 bis 8 Kohlenstoffatomen, für geradkettiges oder verzweigtes Alkenyl mit 3 bis 6 Kohlenstoffatomen, für geradkettiges oder verzweigtes Alkinyl mit 3 bis 5 Kohlenstoffatomen, für Alkoxyalkyl oder Alkoxycarbonylalkyl mit je 1 bis 6 Kohlenstoffatomen im geradkettigen oder verzweigten Alkoxy- und Alkylteil, für Alkoxycarbonyl mit 1 bis 6 Kohlenstoffatomen im geradkettigen oder verzweigten Alkoxyteil, für Carboxyalkyl mit 1 bis 6 Kohlenstoffatomen im geradkettigen oder verzweigten Alkylteil, für Phenylalkyl mit 1 bis 4 Kohlenstoffatomen im geradkettigen oder verzweigten Alkylteil oder für gegebenenfalls ein- bis dreifach, gleich oder verschieden durch Alkyl mit 1 bis 4 Kohlenstoffatomen und Niederalkoxycarbonyl mit 1 bis 4 Kohlenstoffatomen im Alkoxyteil substituiertes Cycloalkyl mit 3 bis 6 Kohlenstoffatomen, oder

R und $R^1$ gemeinsam mit dem Stickstoffatom, an dem sie stehen, einen gesättigten Heterocyclus mit 5 bis 7 Ringgliedern, der durch weitere Heteroatome, wie Sauerstoff oder Stickstoff, unterbrochen sein kann, bilden und

$R^2$ und $R^3$ gleich oder verschieden sind und für geradkettiges oder verzweigtes Alkoxy oder Alkylthio mit je 1 bis 6 Kohlenstoffatomen oder für Phenylthio stehen.

Besonders bevorzugt sind die Verbindungen der Formel (I), in denen

R für Wasserstoff, für geradkettiges oder verzweigtes Alkyl mit 1 bis 6 Kohlenstoffatomen, oder für geradkettiges oder verzweigtes Alkenyl mit 3 oder 4 Kohlenstoffatomen steht,

$R^1$ für Wasserstoff, für geradkettiges oder verzweigtes Alkyl mit 1 bis 6 Kohlenstoffatomen, für geradkettiges oder verzweigtes Alkenyl mit 3 oder 4 Kohlenstoffatomen, für geradkettiges oder verzweigtes Alkinyl mit 3 oder 4 Kohlenstoffatomen, für Alkoxyalkyl mit 1 bis 4 Kohlenstoffatomen je geradkettigem oder verzweigtem Alkoxy- und Alkylteil, für Alkoxycarbonylalkyl mit 1 bis 4 Kohlenstoffatomen je geradkettigem oder verzweigtem Alkoxy- und Alkylteil, für Alkoxycarbonyl mit 1 bis 4 Kohlenstoffatomen im geradkettigen oder verzweigten Alkoxyteil, für Carboxyalkyl mit 1 bis 4 Kohlenstoffatomen im geradkettigen oder verzweigten Alkylteil, für Phenylalkyl mit 1 bis 3 Kohlenstoffatomen im geradkettigen oder verzweigten Alkylteil oder für gegebenenfalls einfach durch Methoxycarbonyl, Ethoxycarbonyl, n-Propoxycarbonyl oder iso-Propoxycarbonyl substituiertes Cycloalkyl mit 3 bis 6 Kohlenstoffatomen steht oder

R und $R^1$ gemeinsam mit dem Stickstoffatom, an dem sie stehen, den Morpholin-, den Piperidin- oder den Piperazinrest bilden, und

$R^2$ und $R^3$ gleich oder verschieden sind und für Methoxy, Ethoxy, Methylthio, Ethylthio, n-Propylthio, iso-Propylthio, n-Butylthio, tert.-Butylthio oder für Phenylthio stehen.

Als Beispiele für die erfindungsgemäß verwendeten Wirkstoffe seien außer den bei den Herstellungsbeispielen genannten aufgezählt:

2-Amino-4,6-bismethoxy-1,3,5-triazin
2-Amino-4,6-bisethoxy-1,3,5-triazin
2-Amino-4,6-bismethylthio-1,3,5-triazin
2-Amino-4,6-bisethylthio-1,3,5-triazin
2-Amino-4,6-bisphenylthio-1,3,5-triazin
2-Amino-4-methoxy-6-methylthio-1,3,5-triazin
2-Amino-4-ethoxy-6-methylthio-1,3,5-triazin
2-Amino-4-ethylthio-6-methylthio-1,3,5-triazin
2-Amino-4-phenylthio-6-methylthio-1,3,5-triazin
2-Amino-4-methoxy-6-ethylthio-1,3,5-triazin
2-Amino-4-ethoxy-6-ethylthio-1,3,5-triazin
2-Amino-4-phenylthio-6-ethylthio-1,3,5-triazin
2-Amino-4-phenylthio-6-methoxy-1,3,5-triazin
2-Amino-4-phenylthio-6-ethoxy-1,3,5-triazin
2-Amino-4,6-bis-tert.-butylthio-1,3,5-triazin

2-Methylamino-4,6-bismethoxy-1,3,5-triazin
2-Methylamino-4,6-bisethoxy-1,3,5-triazin
2-Methylamino-4,6-bismethylthio-1,3,5-triazin
2-Methylamino-4,6-bisethylthio-1,3,5-triazin
2-Methylamino-4,6-bisphenylthio-1,3,5-triazin
2-Methylamino-4-methoxy-6-methylthio-1,3,5-triazin
2-Methylamino-4-ethoxy-6-methylthio-1,3,5-triazin
2-Methylamino-4-ethylthio-6-methylthio-1,3,5-triazin
2-Methylamino-4-phenylthio-6-methylthio-1,3,5-triazin
2-Methylamino-4-methoxy-6-ethylthio-1,3,5-triazin
2-Methylamino-4-ethoxy-6-ethylthio-1,3,5-triazin
2-Methylamino-4-phenylthio-6-ethylthio-1,3,5-triazin
2-Methylamino-4-phenylthio-6-methoxy-1,3,5-triazin
2-Methylamino-4-phenylthio-6-ethoxy-1,3,5-triazin
2-Methylamino-4,6-bis-tert.-butylthio-1,3,5-triazin

2-(N-Allyl-N-carboxymethylamino)-4,6-bismethoxy-1,3,5-triazin,
2-(N-Allyl-N-carboxymethylamino)-4,6-bisethoxy-1,3,5-triazin,
2-(N-Allyl-N-carboxymethylamino)-4,6-bismethylthio-1,3,5-triazin,
2-(N-Allyl-N-carboxymethylamino)-4,6-bisethylthio-1,3,5-triazin,
2-(N-Allyl-N-carboxymethylamino)-4,6-bisphenylthio-1,3,5-triazin,
2-(N-Allyl-N-carboxymethylamino)-4-methoxy-6-methylthio-1,3,5-triazin,
2-(N-Allyl-N-carboxymethylamino)-4-ethoxy-6-methylthio-1,3,5-triazin,

2-(N-Allyl-N-carboxymethylamino)-4-ethylthio-6-methylthio-1,3,5-triazin,
2-(N-Allyl-N-carboxymethylamino)-4-phenylthio-6-methylthio-1,3,5-triazin,
2-(N-Allyl-N-carboxymethylamino)-4-methoxy-6-ethylthio-1,3,5-triazin,
2-(N-Allyl-N-carboxymethylamino)-4-ethoxy-6-ethylthio-1,3,5-triazin,
2-(N-Allyl-N-carboxymethylamino)-4-phenylthio-6-ethylthio-1,3,5-triazin,
2-(N-Allyl-N-carboxymethylamino)-4-phenylthio-6-methoxy-1,3,5-triazin,
2-(N-Allyl-N-carboxymethylamino)-4-phenylthio-6-ethoxy-1,3,5-triazin,
2-(N-Allyl-N-carboxymethylamino)-4,6-bis-tert.-butylthio-1,3,5-triazin,

2-Carboxymethylamino-4,6-bismethoxy-1,3,5-triazin,
2-Carboxymethylamino-4,6-bisethoxy-1,3,5-triazin,
2-Carboxymethylamino-4,6-bismethylthio-1,3,5-triazin,
2-Carboxymethylamino-4,6-bisethylthio-1,3,5-triazin,
2-Carboxymethylamino-4,6-bisphenylthio-1,3,5-triazin,
2-Carboxymethylamino-4-methoxy-6-methylthio-1,3,5-triazin,
2-Carboxymethylamino-4-ethoxy-6-methylthio-1,3,5-triazin,
2-Carboxymethylamino-4-ethylthio-6-methylthio-1,3,5-triazin,
2-Carboxymethylamino-4-phenylthio-6-methylthio-1,3,5-triazin,
2-Carboxymethylamino-4-methoxy-6-ethylthio-1,3,5-triazin,

2-Carboxymethylamino-4-ethoxy-6-ethylthio-1,3,5-triazin,
2-Carboxymethylamino-4-phenylthio-6-ethylthio-1,3,5-triazin,
2-Carboxymethylamino-4-phenylthio-6-methoxy-1,3,5-triazin,
2-Carboxymethylamino-4-phenylthio-6-ethoxy-1,3,5-triazin,
2-Carboxymethylamino-4,6-bis-tert.-butylthio-1,3,5-triazin,

2-(N-Allyl-N-methoxycarbonylamino)-4,6-bismethoxy-1,3,5-triazin,
2-(N-Allyl-N-methoxycarbonylamino)-4,6-bisethoxy-1,3,5-triazin,
2-(N-Allyl-N-methoxycarbonylamino)-4,6-bismethylthio-1,3,5-triazin,
2-(N-Allyl-N-methoxycarbonylamino)-4,6-bisethylthio-1,3,5-triazin,
2-(N-Allyl-N-methoxycarbonylamino)-4,6-bisphenylthio-1,3,5-triazin,
2-(N-Allyl-N-methoxycarbonylamino)-4-methoxy-6-methylthio-1,3,5-triazin,
2-(N-Allyl-N-methoxycarbonylamino)-4-ethoxy-6-methylthio-1,3,5-triazin,
2-(N-Allyl-N-methoxycarbonylamino)-4-ethylthio-6-methylthio-1,3,5-triazin,
2-(N-Allyl-N-methoxycarbonylamino)-4-phenylthio-6-methylthio-1,3,5-triazin,
2-(N-Allyl-N-methoxycarbonylamino)-4-methoxy-6-ethylthio-1,3,5-triazin,
2-(N-Allyl-N-methoxycarbonylamino)-4-ethoxy-6-ethylthio-1,3,5-triazin,

2-(N-Allyl-N-methoxycarbonylamino)-4-phenylthio-6-ethylthio-1,3,5-triazin,
2-(N-Allyl-N-methoxycarbonylamino)-4-phenylthio-6-methox 1,3,5-triazin,
2-(N-Allyl-N-methoxycarbonylamino)-4-phenylthio-6-ethoxy 1,3,5-triazin,
2-(N-Allyl-N-methoxycarbonylamino)-4,6-bis-tert.-butylth 1,3,5-triazin,

2-(N-Allyl-N-ethoxycarbonylamino)-4,6-bismethoxy-1,3,5-triazin,
2-(N-Allyl-N-ethoxycarbonylamino)-4,6-bisethoxy-1,3,5-triazin,
2-(N-Allyl-N-ethoxycarbonylamino)-4,6-bismethylthio-1,3,5-triazin,
2-(N-Allyl-N-ethoxycarbonylamino)-4,6-bisethylthio-1,3,5 triazin,
2-(N-Allyl-N-ethoxycarbonylamino)-4,6-bisphenylthio-1,3,5-triazin,
2-(N-Allyl-N-ethoxycarbonylamino)-4-methoxy-6-methylthio-1,3,5-triazin,
2-(N-Allyl-N-ethoxycarbonylamino)-4-ethoxy-6-methylthio-1,3,5-triazin,
2-(N-Allyl-N-ethoxycarbonylamino)-4-ethylthio-6-methylthio-1,3,5-triazin,
2-(N-Allyl-N-ethoxycarbonylamino)-4-phenylthio-6-methylthio-1,3,5-triazin,
2-(N-Allyl-N-ethoxycarbonylamino)-4-methoxy-6-ethylthio-1,3,5-triazin,
2-(N-Allyl-N-ethoxycarbonylamino)-4-ethoxy-6-ethylthio-1,3,5-triazin,

2-(N-Allyl-N-ethoxycarbonylamino)-4-phenylthio-6-ethylthio-1,3,5-triazin,
2-(N-Allyl-N-ethoxycarbonylamino)-4-phenylthio-6-methoxy-1,3,5-triazin,
2-(N-Allyl-N-ethoxycarbonylamino)-4-phenylthio-6-ethoxy-1,3,5-triazin,
2-(N-Allyl-N-ethoxycarbonylamino)-4,6-bis-tert.-butylthio-1,3,5-triazin,

2-(1-Methyl-1-methoxycarbonylethylamino)-4,6-bismethoxy-1,3,5-triazin,
2-(1-Methyl-1-methoxycarbonylethylamino)-4,6-bisethoxy-1,3,5-triazin,
2-(1-Methyl-1-methoxycarbonylethylamino)-4,6-bismethylthio-1,3,5-triazin,
2-(1-Methyl-1-methoxycarbonylethylamino)-4,6-bisethylthio-1,3,5-triazin,
2-(1-Methyl-1-methoxycarbonylethylamino)-4,6-bisphenylthio-1,3,5-triazin,
2-(1-Methyl-1-methoxycarbonylethylamino)-4-methoxy-6-methylthio-1,3,5-triazin,
2-(1-Methyl-1-methoxycarbonylethylamino)-4-ethoxy-6-methylthio-1,3,5-triazin,
2-(1-Methyl-1-methoxycarbonylethylamino)-4-ethylthio-6-methylthio-1,3,5-triazin,
2-(1-Methyl-1-methoxycarbonylethylamino)-4-phenylthio-6-methylthio-1,3,5-triazin,
2-(1-Methyl-1-methoxycarbonylethylamino)-4-methoxy-6-ethylthio-1,3,5-triazin,

2-(1-Methyl-1-methoxycarbonylethylamino)-4-ethoxy-6-ethylthio-1,3,5-triazin,
2-(1-Methyl-1-methoxycarbonylethylamino)-4-phenylthio-6-ethylthio-1,3,5-triazin,
2-(1-Methyl-1-methoxycarbonylethylamino)-4-phenylthio-6-methoxy-1,3,5-triazin
2-(1-Methyl-1-methoxycarbonylethylamino)-4-phenylthio-6-ethoxy-1,3,5-triazin,
2-(1-Methyl-1-methoxycarbonylethylamino)-4,6-bis-tert.-butylthio-1,3,5-triazin,

2-(1-Methoxycarbonyl-cyclopent-1-yl-amino)-4,6-bismethoxy-1,3,5-triazin,
2-(1-Methoxycarbonyl-cyclopent-1-yl-amino)-4,6-bisethoxy-1,3,5-triazin,
2-(1-Methoxycarbonyl-cyclopent-1-yl-amino)-4,6-bismethylthio-1,3,5-triazin,
2-(1-Methoxycarbonyl-cyclopent-1-yl-amino)-4,6-bisethylthio-1,3,5-triazin,
2-(1-Methoxycarbonyl-cyclopent-1-yl-amino)-4,6-bisphenylthio-1,3,5-triazin,
2-(1-Methoxycarbonyl-cyclopent-1-yl-amino)-4-methoxy-6-methylthio-1,3,5-triazin,
2-(1-Methoxycarbonyl-cyclopent-1-yl-amino)-4-ethoxy-6-methylthio-1,3,5-triazin,
2-(1-Methoxycarbonyl-cyclopent-1-yl-amino)-4-ethylthio-6-methylthio-1,3,5-triazin,
2-(1-Methoxycarbonyl-cyclopent-1-yl-amino)-4-phenylthio-6-methylthio-1,3,5-triazin,

2-(1-Methoxycarbonyl-cyclopent-1-yl-amino)-4-methoxy-6-ethylthio-1,3,5-triazin,
2-(1-Methoxycarbonyl-cyclopent-1-yl-amino)-4-ethoxy-6-ethylthio-1,3,5-triazin,
2-(1-Methoxycarbonyl-cyclopent-1-yl-amino)-4-phenylthio-6-ethylthio-1,3,5-triazin,
2-(1-Methoxycarbonyl-cyclopent-1-yl-amino)-4-phenylthio-6-methoxy-1,3,5-triazin,
2-(1-Methoxycarbonyl-cyclopent-1-yl-amino)-4-phenylthio-6-ethoxy-1,3,5-triazin,
2-(1-Methoxycarbonyl-cyclopent-1-yl-amino)-4,6-bis-tert.-butylthio-1,3,5-triazin,

2-(1-Ethoxycarbonyl-cyclopent-1-yl-amino)-4,6-bismethoxy-1,3,5-triazin,
2-(1-Ethoxycarbonyl-cyclopent-1-yl-amino)-4,6-bisethoxy-1,3,5-triazin,
2-(1-Ethoxycarbonyl-cyclopent-1-yl-amino)-4,6-bismethylthio-1,3,5-triazin,
2-(1-Ethoxycarbonyl-cyclopent-1-yl-amino)-4,6-bisethylthio-1,3,5-triazin,
2-(1-Ethoxycarbonyl-cyclopent-1-yl-amino)-4,6-bisphenylthio-1,3,5-triazin,
2-(1-Ethoxycarbonyl-cyclopent-1-yl-amino)-4-methoxy-6-methylthio-1,3,5-triazin,
2-(1-Ethoxycarbonyl-cyclopent-1-yl-amino)-4-ethoxy-6-methylthio-1,3,5-triazin,
2-(1-Ethoxycarbonyl-cyclopent-1-yl-amino)-4-ethylthio-6-methylthio-1,3,5-triazin,
2-(1-Ethoxycarbonyl-cyclopent-1-yl-amino)-4-phenylthio-6-methylthio-1,3,5-triazin,

2-(1-Ethoxycarbonyl-cyclopent-1-yl-amino)-4-methoxy-6-ethylthio-1,3,5-triazin,
2-(1-Ethoxycarbonyl-cyclopent-1-yl-amino)-4-ethoxy-6-ethylthio-1,3,5-triazin,
2-(1-Ethoxycarbonyl-cyclopent-1-yl-amino)-4-phenylthio-6-ethylthio-1,3,5-triazin,
2-(1-Ethoxycarbonyl-cyclopent-1-yl-amino)-4-phenylthio-6-methoxy-1,3,5-triazin,
2-(1-Ethoxycarbonyl-cyclopent-1-yl-amino)-4-phenylthio-6-ethoxy-1,3,5-triazin,
2-(1-Ethoxycarbonyl-cyclopent-1-yl-amino)-4,6-bis-tert.-butylthio-1,3,5-triazin,

2-(N-Allylamino)-4-methoxy-6-thiomethyl-1,3,5-triazin,
2-(N-Allylamino)-4,6-bismethoxy-1,3,5-triazin,
2-(N-n-Propyl)-4,6-bismethoxy-1,3,5-triazin und
2-(N-n-Butylamino)-4,6-bismethoxy-1,3,5-triazin.

Einzelne der erfindungsgemäß zu verwendenden Wirkstoffe sind neu. Die bekannten Verbindungen können nach literaturbekannten Verfahren hergestellt werden, ebenso wie die neuen Triazin-Derivate der Formel (Ia)

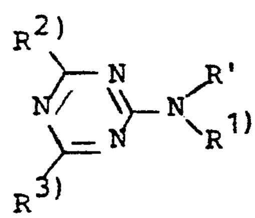

(Ia)

in welcher R', $R^{1)}$, $R^{2)}$ und $R^{3)}$ die unten angegebene Bedeutung haben, indem man

a) 2-Halogentriazine der Formel (II)

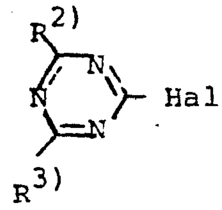

(II)

in welcher

Hal für ein Halogenatom, vorzugsweise Chlor, steht und

$R^{2)}$ und $R^{3)}$ die unten angegebene Bedeutung haben,

mit Aminen der Formel (III)

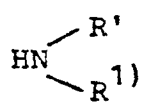

(III)

in welcher

R' und $R^{1)}$ die unten angegebene Bedeutung haben,

gegebenenfalls in Gegenwart eines Lösungs- oder Verdünnungsmittels und gegebenenfalls in Gegenwart eines Säurebindemittels umsetzt oder

b) 2-Aminotriazine der Formel (IV)

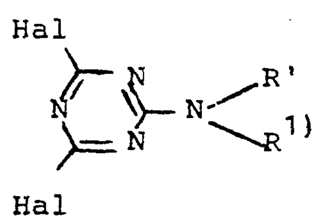

(IV)

0174574

in welcher

Hal für Halogen, vorzugsweise Chlor, steht und

R' und $R^{1)}$ die weiter unten angegebene Bedeutung haben,

mit Verbindungen der Formel (V) im 1. Schritt

$R^{2)}H$ (V)

und gegebenenfalls mit Verbindungen der Formel (VI) im 2. Schritt

$R^{3)}H$ (VI)

wobei

$R^{2)}$ und $R^{3)}$ die weiter unten angegebene Bedeutung haben,

gegebenenfalls in Gegenwart eines Lösungs- oder Verdünnungsmittels, gegebenenfalls in Gegenwart eines Säurebindemittels umsetzt. Im Falle, daß $R^{2)}$ mit $R^{3)}$ identisch ist, können beide Reste in einem Schritt eingeführt werden oder

c) Verbindungen der Formel (VII)

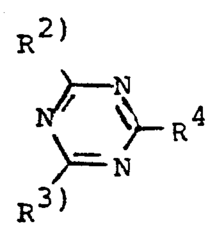

(VII)

worin

$R^4$ für Alkylthio steht, und alle Reste $R^{2)}$, $R^{3)}$ und $R^4$ identisch sind,

mit stöchiometrischen Mengen an Aminen der Formel (III) gegebenenfalls in Gegenwart eines Verdünnungs- oder Lösungsmittels bei Temperaturen zwischen 30°C und 200°C umsetzt und eine der Gruppen selektiv austauscht.

In der Formel (Ia) haben die Substituenten die folgende Bedeutung:

R' steht für Wasserstoff oder Alkenyl,

$R^{1)}$ steht für Alkyl mit mehr als 3 Kohlenstoffatomen, Alkenyl, Alkinyl, Alkoxyalkyl, Alkoxycarbonylalkyl, Carboxyalkyl und

$R^{2)}$ und $R^{3)}$ gleich oder verschieden sind und für Alkylthio stehen.

In der Formel (Ia) stehen vorzugsweise

R' für Wasserstoff oder für geradkettiges oder verzweigtes Alkenyl mit 3 bis 6 Kohlenstoffatomen,

$R^{1)}$ für geradkettiges oder verzweigtes Alkyl mit 4 bis 8 Kohlenstoffatomen, für geradkettiges oder verzweigtes Alkenyl mit 3 bis 6 Kohlenstoffatomen,

für geradkettiges oder verzweigtes Alkinyl mit 3 bis 5 Kohlenstoffatomen, für Alkoxyalkyl oder Alkoxycarbonylalkyl mit je 1 bis 6 Kohlenstoffatomen im geradkettigen oder verzweigten Alkoxy- und Alkylteil oder für Carboxyalkyl mit 1 bis 6 Kohlenstoffatomen im geradkettigen oder verzweigten Alkylteil, und

$R^{2)}$ und $R^{3)}$ gleich oder verschieden sind und für geradkettiges oder verzweigtes Alkylthio mit 1 bis 6 Kohlenstoffatomen stehen.

Besonders bevorzugt sind die Verbindungen der Formel (Ia), in denen

R' für Wasserstoff oder für geradkettiges oder verzweigtes Alkenyl mit 3 oder 4 Kohlenstoffatomen steht,

$R^{1)}$ für geradkettiges oder verzweigtes Alkyl mit 4 bis 6 Kohlenstoffatomen, für geradkettiges oder verzweigtes Alkenyl mit 3 oder 4 Kohlenstoffatomen, für geradkettiges oder verzweigtes Alkinyl mit 3 oder 4 Kohlenstoffatomen, für Alkoxyalkyl mit 1 bis 4 Kohlenstoffatomen je geradkettigem oder verzweigtem Alkoxy- und Alkylteil, für Alkoxycarbonylalkyl mit 1 bis 4 Kohlenstoffatomen je geradkettigem oder verzweigtem Alkoxy- und Alkylteil oder für Carboxyalkyl mit 1 bis 4 Kohlenstoffatomen im geradkettigen oder verzweigten Alkylteil steht, und

$R^{2)}$ und $R^{3)}$ gleich oder verschieden sind und für Methylthio, Ethylthio, n-Propylthio, iso-Propylthio, n-Butylthio oder tert.-Butylthio stehen.

Die zur Durchführung des erfindungsgemäßen Verfahrens als Ausgangsstoffe benötigten 2-Halogentriazine sind durch die Formel (II) definiert. In dieser Formel (II) stehen $R^{2)}$ und $R^{3)}$ für diejenigen Reste, die schon bei der Beschreibung der erfindungsgemäßen Stoffe der Formel (Ia) genannt wurden.

Die 2-Halogentriazine der Formel (II) sind größtenteils bekannt und können nach gängigen Verfahren, z.B. aus Trichlortriazin durch schrittweisen Austausch der Chloratome hergestellt werden (vgl. J. R. Dudley et. al., Journal American Chemical Society 73, 2986 (1951)).

Die weiterhin zur Durchführung des erfindungsgemäßen Verfahrens als Ausgangsstoffe benötigten Amine sind durch die Formel (III) allgemein definiert. In dieser Formel stehen die Reste R' und $R^{1)}$ für diejenigen Reste, die schon bei der Beschreibung der erfindungsgemäßen Stoffe der Formel (Ia) für diese Substituenten genannt wurden.

Die Amine sind allgemein bekannte Verbindungen der organischen Chemie und käuflich zu erwerben.

Weiterhin werden bei dem erfindungsgemäßen Verfahren b) 2-Aminotriazine benötigt, die durch die Formel (IV) definiert sind. In dieser Formel stehen die Reste R' und $R^{1)}$ für diejenigen Substituenten, die schon bei der Beschreibung der erfindungsgemäßen Stoffe der Formel (Ia) genannt wurden.

Die Verbindungen sind größtenteils bekannt und können nach gängigen Verfahren hergestellt werden (vgl. H. Koopman u. J. Daams, "Rec. Trav. Chim. 77, 235 (1958); Brit. Pat. 814.949).

Die weiterhin als Ausgangsstoffe benötigten Verbindungen der Formeln (V) und (VI) sind allgemein in der organischen Chemie bekannte Verbindungen, wie Alkohole und Thioalkohole, und käuflich zu erwerben.

Die bei Variante c) einzusetzenden Verbindungen der Formel (VII) sind bekannt /vgl. J. Dudley et. al "J. Am. Chem. Soc." 73, 2986 (1951); A. Hofmann" Ber." 18, 2196 (1985)7.

Als Verdünnungsmittel zur Durchführung der erfindungsgemäßen Verfahren kommen inerte organische Lösungsmittel in Frage.

Hierzu gehören insbesondere aliphatische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff; Ether, wie Diethylether, Dioxan, Tetrahydrofuran oder Ethylenglykoldimethyl- oder -diethylether; Ketone, wie Aceton oder Butanon; Nitrile, wie Acetonitril oder Propionitril; Amide, wie Dimethylformamid, Dimethylacetamid, N-Methylformanilid, N-Methylpyrrolidon oder Hexamethylphosphorsäuretriamid oder Ester, wie Essigsäureethylester.

Die erfindungsgemäßen Verfahren werden gegebenenfalls in Gegenwart eines Säurebindemittels durchgeführt.

Als solche kommen alle üblichen anorganischen oder organischen Basen in Frage. Hierzu gehören beispielsweise Alkalimetallhydroxide, -amide, -alkoholate oder -hydride, wie Natriumhydroxid oder Kaliumhydroxid, Natriummethylat oder Kalium-t-butylat, Natriumhydrid oder Natriumamid, Alkalimetallcarbonate, wie Natriumcarbonat, Kaliumcarbonat oder Natriumhydrogencarbonat, sowie tertiäre Amine, wie Triethylamin, N,N-Dimethylanilin, Pyridin oder N,N-Dimethylaminopyridin.

Die Reaktionstemperaturen können bei der Durchführung der erfindungsgemäßen Verfahren (a) und (b) in einem größeren Bereich variiert werden. Im allgemeinen arbeitet man bei Temperaturen zwischen 0°C und +200°C, vorzugsweise bei Temperaturen zwischen 50°C bis 120°C.

Bei der Verfahrensvariante (c) wird bei Temperaturen zwischen 30°C und 200°C, vorzugsweise zwischen 60°C und 170°C, gearbeitet.

Bei den Umsetzungen (a) und (b) können die Einzelschritte in beliebiger Reihenfolge durchgeführt werden, es kann erst ein Rest, z.B. $R^{2)}$, dann der Aminorest und dann der Rest $R^{3)}$ eingeführt werden.

Die Isolierung der Verbindungen der Formel (Ia) erfolgt nach üblichen Methoden.

Die erfindungsgemäß verwendbaren Wirkstoffe weisen eine fungizide Wirkung auf, vor allem eine starke Wirkung gegen BCM-resistente Pilzstämme. Sie können deshalb eingesetzt werden zur Bekämpfung von unerwünschten Mikroorganismen. Die Wirkstoffe sind für den Gebrauch als Fungizide geeignet, besonders in Kombination mit anderen fungiziden Wirkstoffen zur Komplettierung des Wirkungsspektrums und vor allem zur Erfassung der sensiblen und resistenten Pilzstämme.

Die Wirkstoffe können vor allem gegen alle BCM-resistenten Stämme, die vorzugsweise bei echten Mehltaupilzen, Schorfpilzen, Cercospora-Arten, Mycosphaerella-Arten, Botrytis-Arten, Pseudocercosporella-Arten, Penicillium-Arten, Fusarium-Arten, Sclerotinia-Arten, Cladosporium-Arten, Colletotrichum-Arten und Verticillium-Arten beobachtet werden, eingesetzt werden.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Die erfindungsgemäßen Wirkstoffe besitzen daneben auch eine wurzelsystemische Wirkung gegen Blattinsekten,

erwähnt sei außerdem die fungizide Wirkung gegen Pyricularia oryzae am Reis (protektiv und systemisch). Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier- und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycol-ether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäß zu verwendenden Wirkstoffe können in den Formulierungen in Mischung mit anderen bekannten Wirkstoffen vorliegen, wie Fungizide, Insektizide, Akarizide und Herbizide, sowie in Mischungen mit Düngemitteln und Wachstumsregulatoren.

Die zu verwendenden Wirkstoffe können als solche, in Form ihrer Formulierungen oder den daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen,

Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 %, am Wirkungsort erforderlich.

Die vorzugsweise Ausführungsform in der Praxis sieht vor, daß mindestens ein Triazinderivat der Formel (I) in Mischung mit einem fungiziden Benzimidazolcarbamat, wie z.B. BCM und/oder einer vor, während oder nach der Applikation BCM-bildenden Verbindung, wie z.B. einer Verbindung aus der Gruppe der Phenylendiaminthioharnstoffcarbamate, in Mischung angewendet wird, die wie üblich formuliert werden kann. Beispiele für die Gruppe der BCM-bildenden Verbindungen sind :

Methyl-1-(butylcarbamoyl)-benzimidazol-2-ylcarbamat, 2-(2-Furyl)-benzimidazol, 2-(4-Thiazolyl)-benzimidazol, Methylbenzimidazol-2-ylcarbamat, 1,2-Bis-(3-methoxycarbonyl-2-thioureido)-phenyl, 1,2-Bis-(3-ethoxycarbonyl-2-thioureido)-phenyl, 2-(O,S-Dimethylphosphorylamino)-1-(3'-methoxy-carbonyl-2'-thioureido)-phenyl oder 2-(O,O-Dimethylthiophosphorylamino)-1-(3'-methoxycarbonyl-2'-thioureido)-phenyl.

Diese Ausführungsform ist zur gleichzeitigen Erfassung der sensiblen und resistenten Pilzstämme wichtig.

Verwendungsbeispiele

In den nachfolgenden Verwendungsbeispielen wird die nachfolgend angegebene Verbindung als Vergleichssubstanz eingesetzt:

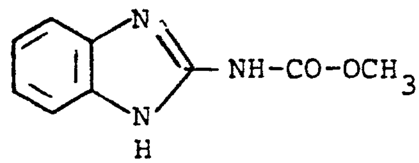

Methyl-benzimidazol-2-yl-carbamat (BCM)

Beispiel A

Botrytis-Test (Bohne)/protektiv/Botrytis cinerea-BCMresistent

Lösungsmittel: 4,7 Gewichtsteile Aceton
Emulgator: 0,3 Gewichtsteile Alkyl-aryl-Polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur

Tropfnässe. Nach Antrocknen des Spritzbelages werden auf jedes Blatt 2 kleine mit Botrytis cinerea bewachsene Agarstückchen aufgelegt. Die inokulierten Pflanzen werden in einer abgedunkelten, feuchten Kammer bei 20°C aufgestellt. 3 Tage nach der Inokulation wird die Größe der Befallsflecken auf den Blättern ausgewertet.

Eine deutliche Überlegenheit in Wirksamkeit gegenüber dem Stand der Technik zeigen in diesem Test z.B. die Verbindungen gemäß folgender Beispiele:

Tabelle A

Botrytis-Test (Bohne)/protektiv/ Botrytis cinerea
BCM-resistent

| Wirkstoffe | Befall in % bei einer Wirkstoffkonzentration von 100 ppm |
|---|---|
| 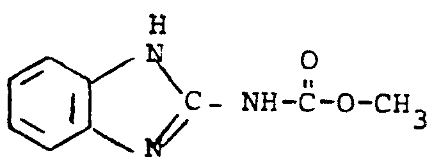 (bekannt) | 100 |
| Erfindungsgemäß: | |
| 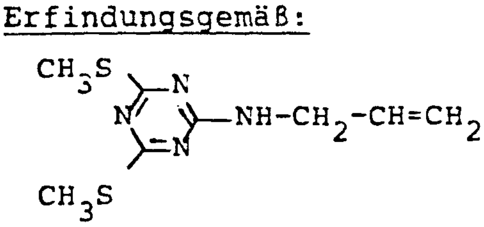 | 7 |

Beispiel B

Botrytis cinerea / radiales Myzelwachstum

In einen Agar, der aus Glucose, Malzextrakt und Pepton hergestellt wird, werden erfindungsgemäße Verbindungen jeweils in abgestuften Konzentrationen eingearbeitet. Zur Herstellung einer zweckmäßigen Wirkstoffzubereitung wird der Wirkstoff in Aceton gelöst. Das Mengenverhältnis von Aceton zu Nährboden beträgt 0,2 : 99,8. Nach dem Erstarren des Agars erfolgt die Inokulation der so hergestellten Agarproben mit frischem Myzel von Botrytris cinerea - Reinkulturen, die auf Benzimidazol-Fungizide entweder sensibel oder resistent reagieren.

Nach dreitägiger Inkubation bei 25°C wird das radiale Myzelwachstum ausgewertet. In der Tabelle B ist als $ED_{50}$-Wert diejenige Konzentration des Wirkstoffes angegeben, bei der das Myzelwachstum zu 50 % inhibiert ist.

Eine deutliche Überlegenheit in der Wirksamkeit gegenüber dem Stand der Technik, bezogen auf pilzliche Isolate mit Resistenz gegen Benzimidazol-Fungizide, zeigen in diesem Test z.B. Verbindungen gemäß folgender Beispiele:

Tabelle B

Botrytis cinerea / radiales Myzelwachstum

| Wirkstoffe | $ED_{50}$-Wert ermittelt an einem sensiblen Isolat (ppm) | $ED_{50}$-Wert ermittelt an einem resistenten Isolat (ppm) |
|---|---|---|
| 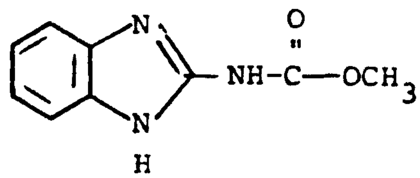 (bekannt) | 0,06 | > 100 |
| 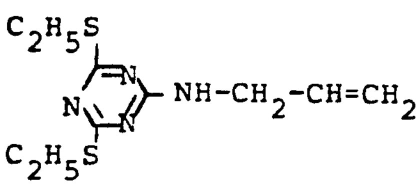 | > 100 | 1,8 |
| 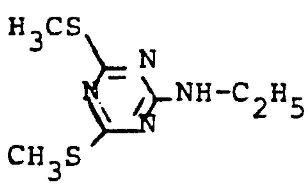 | > 100 | 6 |
| 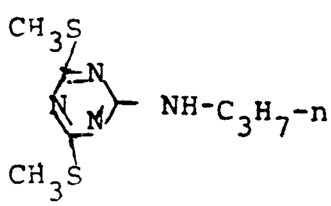 | > 100 | 0,8 |
| 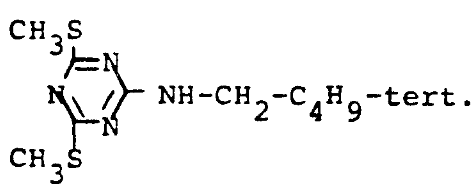 | > 100 | 50 |
| 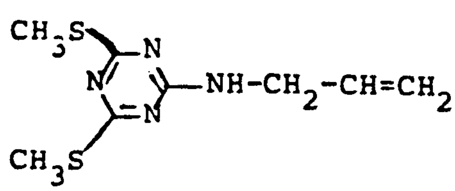 | > 100 | 2,0 |

Tabelle B (Fortsetzung)

Botrytis cinerea / radiales Myzelwachstum

| Wirkstoffe | $ED_{50}$-Wert ermittelt an einem sensiblen Isolat (ppm) | $ED_{50}$-Wert ermittelt an einem resistenten Isolat (ppm) |
|---|---|---|
| 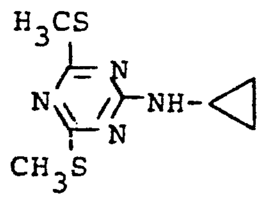 | 90 | 9 |
| 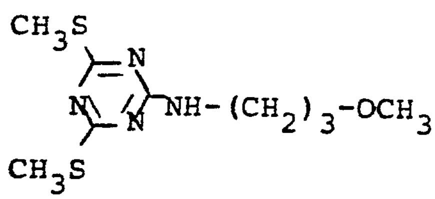 | > 100 | 11 |
| 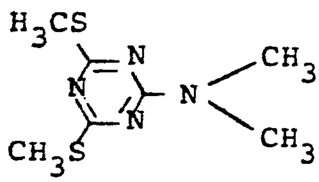 | > 100 | 5 |
| 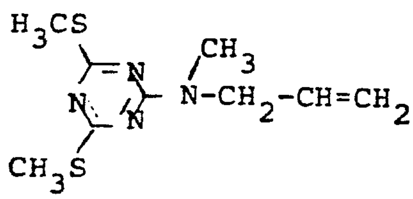 | >100 | 7,3 |

Beispiel C

Pseudocercosporella herpotrichoides /radiales Myzelwachstum

In einen Agar, der aus Glucose, Malzextrakt und Pepton hergestellt wird, werden erfindungsgemäße Verbindungen jeweils in abgestuften Konzentrationen eingearbeitet. Zur Herstellung einer zweckmäßigen Wirkstoffzubereitung wird der Wirkstoff in Aceton gelöst. Das Mengenverhältnis von Aceton zu Nährboden beträgt 0,2 : 99,8. Nach dem Erstarren des Agars erfolgt die Inokulation der so hergestellten Agarproben mit Sporen von Pseudocercosporella herpotrichoides - Reinkulturen, die auf Benzimidazol-Fungizide entweder sensibel oder resistent reagieren.

Nach dreiwöchiger Inkubation bei 25°C wird das radiale Wachstum ausgewertet. In der Tabelle C ist als $ED_{50}$-Wert diejenige Konzentration des Wirkstoffs angegeben, bei der das Wachstum zu 50 % inhibiert ist.

Eine deutliche Überlegenheit in der Wirksamkeit gegenüber dem Stand der Technik, bezogen auf pilzliche Isolate mit Resistenz gegen Benzimidazol-Fungizide, zeigt in diesem Test die folgende Verbindung:

Tabelle C

Pseudocercosporella herpotrichoides / radiales Myzelwachstum

| Wirkstoffe | $ED_{50}$-Wert ermittelt an einem sensiblen Isolat (ppm) | $ED_{50}$-Wert ermittelt an einem resistenten Isolat (ppm) |
|---|---|---|
| 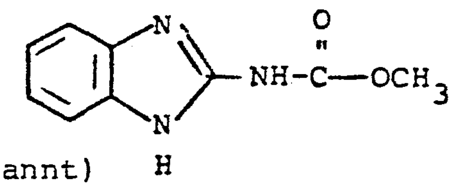 (bekannt) | 0,1 | 100 |
| 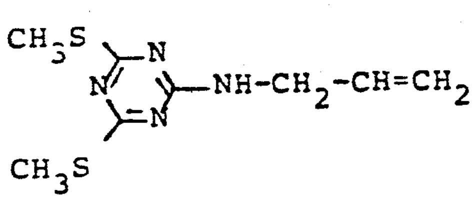 | 100 | 0,4 |

0174574

Herstellungsbeispiele

Beispiel 1

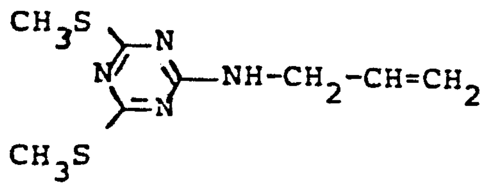

10 g (0,05 mol) 2-Allylamino-4,6-dichlor-1,3,5-triazin werden in 200 ml Dioxan gelöst. Hierzu tropft man 50 ml 2n-Natriummethylmercaptid-Lösung und erhitzt 3 Stunden zum Sieden. Das Reaktionsgemisch wird zu 1 l Wasser gegeben und mit Dichlormethan extrahiert. Nachdem die organische Phase über Natriumsulfat getrocknet und das Lösungsmittel im Vakuum entfernt wird, erhält man nach Umkristallisieren aus Waschbenzin 6.5 g (58 % der Theorie) an 2-Allylamino-4,6-bismethylthio-1,3,5-triazin mit einem Schmelzpunkt von 108°C.

Beispiel 2

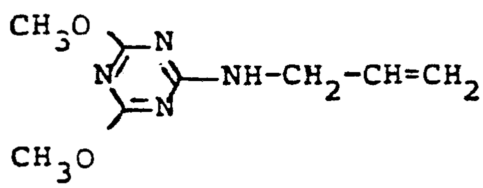

Zu 9 g (0.05 mol) 2-Chlor-4,6-bismethoxy-1,3,5-triazin in 150 ml Dioxan tropft man bei Raumtemperatur nacheinander 2.9 g (0.05 mol) Allylamin und 5.15 g (0.05 mol) Triethylamin und erhitzt 6 Stunden zum Sieden. Nach der Zugabe von 1 l Wasser, Extraktion mit Dichlormethan und Entfernen des Lösungsmittels wird der resultierende

Rückstand aus Ethanol umkristallisiert. Man erhält 6 g (60 % der Theorie) 2-Allylamino-4,6-bismethoxy-1,3,5-triazin mit einem Schmelzpunkt von 82°C.

Analog den vorbeschriebenen Beispielen werden die folgenden Verbindungen der Formel (I) hergestellt:

| Bsp. Nr. | R | $R^1$ | $R^2$ | $R^3$ | Physikal. Konstanten (Schmelzpunkt: °C; Brechungsindex: $n_D^{20}$) |
|---|---|---|---|---|---|
| 3 | H | H | $-SCH_3$ | $-SCH_3$ | 192-195 |
| 4 | H | $-CH_3$ | $-SCH_3$ | $-SCH_3$ | 160-162 |
| 5 | H | $-C_2H_5$ | $-SCH_3$ | $-SCH_3$ | 110-113 |
| 6 | H | $-C_3H_7$-n | $-SCH_3$ | $-SCH_3$ | 117 |
| 7 | H | $-C_3H_7$-iso | $-SCH_3$ | $-SCH_3$ | Öl |
| 8 | H | $-(CH_2)_3-OCH_3$ | $-SCH_3$ | $-SCH_3$ | 64 |
| 9 | H | $-(CH_2)_3-OC_3-H_7$-iso | $-SCH_3$ | $-SCH_3$ | 67-70 |
| 10 | H | $-C_4H_9$-n | $-SCH_3$ | $-SCH_3$ | 85-86 |
| 11 | H | $-C_4H_9$-t | $-SCH_3$ | $-SCH_3$ | Öl |
| 12 | H | $-CH_2-C_4H_9$-t | $-SCH_3$ | $-SCH_3$ | 93-96 |
| 13 | H | $-C(CH_3)_2-C_2H_5$ | $-SCH_3$ | $-SCH_3$ | Öl |
| 14 | H | 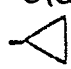 | $-SCH_3$ | $-SCH_3$ | 77 |
| 15 | H | 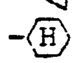 | $-SCH_3$ | $-SCH_3$ | 86-88 |
| 16 | $-CH_3$ | $-CH_3$ | $-SCH_3$ | $-SCH_3$ | 135 |

| Bsp. Nr. | R | $R^1$ | $R^2$ | $R^3$ | Physikal. Konstanten (Schmelzpunkt: °C; Brechungsindex: $n_D^{20}$) |
|---|---|---|---|---|---|
| 17 | $-CH_3$ | $-CH_2-CH=CH_2$ | $-SCH_3$ | $-SCH_3$ | 35-37 |
| 18 | $-CH_2-CH=CH_2$ | $-CH_2-CH=CH_2$ | $-SCH_3$ | $-SCH_3$ | $<$ 40 |
| 19 | H | $-CH_2-$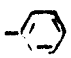 | $-SCH_3$ | $-SCH_3$ | 144 |
| 20 | H | $-CH-$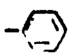 $CH_3$ | $-SCH_3$ | $-SCH_3$ | 1.6071 |
| 21 | H | $-CH_2-CH=CH_2$ | $-SC_2H_5$ | $-SC_2H_5$ | 115 |
| 22 | H | $-CH_2-CH=CH_2$ | $-SC_4H_9-t$ | $-SC_4H_9-t$ | 56-57 |
| 23 | H | $-CH_2-CH=CH_2$ | $-S-$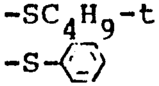 | $-S-$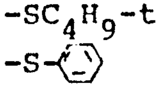 | 58-60 |
| 24 | $-CH_2-CH=CH_2$ | $-CH_2-COOH$ | $-SCH_3$ | $-SCH_3$ | 120-123 |
| 25 | H | $-CH_2-COOH$ | $-SCH_3$ | $-SCH_3$ | 223-226 |
| 26 | $-CH_2-CH=CH_2$ | $-CH_2-COOC_2H_5$ | $-SCH_3$ | $-SCH_3$ | 58-60 |
| 27 | H | $-C(CH_3)_2-COOCH_3$ | $-SCH_3$ | $-SCH_3$ | 128-132 |
| 28 | H | 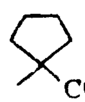 $COOC_2H_5$ | $-SCH_3$ | $-SCH_3$ | 129-134 |

| Bsp. Nr. | R | $R^1$ | $R^2$ | $R^3$ | Physikal. Konstanten (Schmelzpunkt: °C; Brechungsindex: $n_D^{20}$) |
|---|---|---|---|---|---|
| 29 | H | $-CH_2-CH=CH_2$ | $-OC_2H_5$ | $-OC_2H_5$ | 104 |
| 30 | H | $-(CH_2)_5-CH_3$ | $-SCH_3$ | $-SCH_3$ | 78-80 |
| 31 | H | $-CH_2-C\equiv CH$ | $-SCH_3$ | $-SCH_3$ | 178 |
| 32 | H | $-COOC_3H_7-i$ | -SCH3 | $-SCH_3$ | 84 |
| 33 | H | $-COOC_3H_7-i$ | $-OCH_3$ | $-OCH_3$ | 87 |
| 34 | H | $-COOC_3H_7-i$ | $-SC_2H_5$ | $-SC_2H_5$ | 1.5722 |
| 35 | H | $-COOC_3H_7-i$ | $-OC_2H_5$ | $-OC_2H_5$ | 1.5330 |
| 36 | H | $-COOC_2H_5$ | $-SCH_3$ | $-SCH_3$ | 94 |
| 37 | H | $-COOC_2H_5$ | $-SC_2H_5$ | $-SC_2H_5$ | 1.5861 |
| 38 | H | $-COOC_4H_9-t$ | $-SCH_3$ | $-SCH_3$ | 117 |
| 39 | H | $-COOC_4H_9-n$ | $-SCH_3$ | $-SCH_3$ | 1.5548 |
| 40 | H | $-COOC_3H_7-n$ | $-SCH_3$ | $-SCH_3$ | 70 |
| 41 | H | $-COOCH_3$ | $-SCH_3$ | $-SCH_3$ | 178 |

Patentansprüche:

1. Fungizide Mittel, gekennzeichnet durch einen Gehalt an mindestens einem Triazin-Derivat der Formel (I)

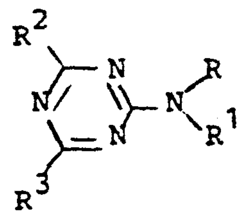

(I)

in welcher

R für Wasserstoff, Alkyl oder Alkenyl steht,

$R^1$ für Wasserstoff, Alkyl, Alkenyl, Alkinyl, Alkoxyalkyl, Alkoxycarbonyl, Alkoxycarbonylalkyl, Carboxyalkyl, Aralkyl oder gegebenenfalls ein- bis mehrfach, gleich oder verschieden substituiertes Cycloalkyl steht oder

R und $R^1$ gemeinsam mit dem Stickstoffatom, an dem sie stehen, einen gesättigten Heterocyclus bilden, der weitere Heteroatome enthalten kann und

$R^2$ und $R^3$ gleich oder verschieden sind und für Alkoxy, Alkylthio oder Phenylthio stehen.

2. Fungizide Mittel nach Anspruch 1, wobei

R für Wasserstoff, für geradkettiges oder verzweigtes Alkyl mit 1 bis 8 Kohlenstoffatomen oder für geradkettiges oder verzweigtes Alkenyl mit 3 bis 6 Kohlenstoffatomen steht,

$R^1$ für Wasserstoff, für geradkettiges oder verzweigtes Alkyl mit 1 bis 8 Kohlenstoffatomen, für geradkettiges oder verzweigtes Alkenyl mit 3 bis 6 Kohlenstoffatomen, für geradkettiges oder verzweigtes Alkinyl mit 3 bis 5 Kohlenstoffatomen, für Alkoxyalkyl oder Alkoxycarbonylalkyl mit je 1 bis 6 Kohlenstoffatomen im geradkettigen oder verzweigten Alkoxy- und Alkylteil, für Alkoxycarbonyl mit 1 bis 6 Kohlenstoffatomen im geradkettigen oder verzweigten Alkoxyteil, für Carboxyalkyl mit 1 bis 6 Kohlenstoffatomen im geradkettigen oder verzweigten Alkylteil, für Phenylalkyl mit 1 bis 4 Kohlenstoffatomen im geradkettigen oder verzweigten Alkylteil oder für gegebenenfalls ein- bis dreifach, gleich oder verschieden durch Alkyl mit 1 bis 4 Kohlenstoffatomen und Niederalkoxycarbonyl mit 1 bis 4 Kohlenstoffatomen im Alkoxyteil substituiertes Cycloalkyl mit 3 bis 6 Kohlenstoffatomen steht oder

R und $R^1$ gemeinsam mit dem Stickstoffatom, an dem sie stehen, einen gesättigten Heterocyclus mit 5 bis 7 Ringgliedern bilden, der weitere Sauerstoff- oder Stickstoffatome enthalten kann, und

$R^2$ und $R^3$ gleich oder verschieden sind und für geradkettiges oder verzweigtes Alkoxy oder Alkylthio mit je 1 bis 6 Kohlenstoffatomen oder für Phenylthio stehen.

3. Fungizide Mittel gemäß Anspruch 1, wobei

R für Wasserstoff, für geradkettiges oder verzweigtes Alkyl mit 1 bis 6 Kohlenstoffatomen oder für geradkettiges oder verzweigtes Alkenyl mit 3 oder 4 Kohlenstoffatomen steht,

$R^1$ für Wasserstoff, für geradkettiges oder verzweigtes Alkyl mit 1 bis 6 Kohlenstoffatomen, für geradkettiges oder verzweigtes Alkenyl mit 3 oder 4 Kohlenstoffatomen, für geradkettiges oder verzweigtes Alkinyl mit 3 oder 4 Kohlenstoffatomen, für Alkoxyalkyl mit 1 bis 4 Kohlenstoffatomen je geradkettigem oder verzweigten Alkoxy- und Alkylteil, für Alkoxycarbonylalkyl mit 1 bis 4 Kohlenstoffatomen je geradkettigem oder verzweigten Alkoxy- und Alkylteil, für Alkoxycarbonyl mit 1 bis 4 Kohlenstoffatomen im geradkettigen oder verzweigten Alkoxyteil, für Carboxyalkyl mit 1 bis 4 Kohlenstoffatomen im geradkettigen oder verzweigten Alkylteil, für Phenylalkyl mit 1 bis 3 Kohlenstoffatomen im geradkettigen oder verzweigten Alkylteil oder für gegebenenfalls einfach durch Methoxycarbonyl, Ethoxycarbonyl, n-Propoxycarbonyl

oder iso-Propoxycarbonyl substituiertes Cycloalkyl mit 3 bis 6 Kohlenstoffatomen steht oder

R und $R^1$ gemeinsam mit dem Stickstoffatom, an dem sie stehen, einen Piperidin-, Morpholin- oder Piperazinrest bilden, und

$R^2$ und $R^3$ gleich oder verschieden sind und für Methoxy, Ethoxy, Methylthio, Ethylthio, n-Propylthio, iso-Propylthio, n-Butylthio, tert.-Butylthio oder für Phenylthio stehen.

4. Verfahren zur Bekämpfung von Pilzen, dadurch gekennzeichnet, daß man Triazin-Derivate der Formel (I) gemäß Anspruch 1 auf Pilze und/oder ihren Lebensraum einwirken läßt.

5. Verwendung von Triazin-Derivaten der Formel (I) gemäß Anspruch 1 zur Bekämpfung von Pilzen.

6. Verfahren zur Herstellung von fungiziden Mitteln, dadurch gekennzeichnet, daß man Triazin-Derivate der Formel (I) gemäß Anspruch 1 mit Streckmitteln und/oder oberflächenaktiven Mitteln vermischt.

7. Verfahren zur Herstellung von fungiziden Mitteln gemäß Ansprüchen 1 und 6, dadurch gekennzeichnet, daß man Triazin-Derivate der Formel (I) mit Benzimidazolcarbamaten und/oder BCM-bildenden Verbindungen und mit Streckmitteln und/oder oberflächenaktiven Mitteln vermischt.

8. Triazin-Derivate der Formel (Ia)

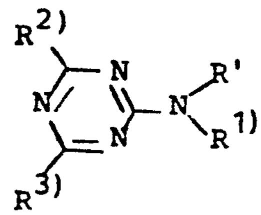

(Ia)

in welcher

R' für Wasserstoff oder Alkenyl steht,

$R^{1)}$ für Alkyl mit mehr als 3 Kohlenstoffatomen, Alkenyl, Alkinyl, Alkoxyalkyl, Alkoxycarbonylalkyl, Carboxyalkyl steht und

$R^{2)}$ und $R^{3)}$ gleich oder verschieden sind und für Alkylthio stehen.

9. Triazin-Derivate der Formel (Ia) gemäß Anspruch 8, worin

R' für Wasserstoff oder für geradkettiges oder verzweigtes Alkenyl mit 3 bis 6 Kohlenstoffatomen,

$R^{1)}$ für geradkettiges oder verzweigtes Alkyl mit 4 bis 8 Kohlenstoffatomen, für geradkettiges oder verzweigtes Alkenyl mit 3 bis 6 Kohlenstoffatomen, für geradkettiges oder verzweigtes Alkinyl mit 3 bis 5 Kohlenstoffatomen, für Alkoxyalkyl oder Alkoxycarbonylalkyl mit je 1 bis 6 Kohlenstoffatomen im geradkettigen oder verzweigten Alkoxy- und Alkylteil, für Carboxyalkyl mit 1 bis 6 Kohlenstoffatomen im geradkettigen oder verzweigten Alkylteil stehen und

$R^{2)}$ und $R^{3)}$ gleich oder verschieden sind und für geradkettiges oder verzweigtes Alkylthio mit 1 bis 6 Kohlenstoffatomen stehen.

10. Triazin-Derivate der Formel (Ia) gemäß Anspruch 8, wobei

R' für Wasserstoff oder für geradkettiges oder verzweigtes Alkenyl mit 3 oder 4 Kohlenstoffatomen steht,

$R^{1)}$ für geradkettiges oder verzweigtes Alkyl mit 4 bis 6 Kohlenstoffatomen, für geradkettiges oder verzweigtes Alkenyl mit 3 oder 4 Kohlenstoffatomen, für geradkettiges oder verzweigtes Alkinyl mit 3 oder 4 Kohlenstoffatomen, für

Alkoxyalkyl mit 1 bis 4 Kohlenstoffatomen je geradkettigem oder verzweigten Alkoxy- und Alkylteil, für Alkoxycarbonylalkyl mit 1 bis 4 Kohlenstoffatomen je geradkettigem oder verzweigten Alkoxy- und Alkylteil oder für Carboxyalkyl mit 1 bis 4 Kohlenstoffatomen im geradkettigen oder verzweigten Alkylteil steht, und

$R^{2)}$ und $R^{3)}$ gleich oder verschieden sind und für Methylthio, Ethylthio, n-Propylthio, iso-Propylthio, n-Butylthio oder tert.-Butylthio stehen.

11. Verfahren zur Herstellung von Triazin-Derivaten der Formel (Ia)

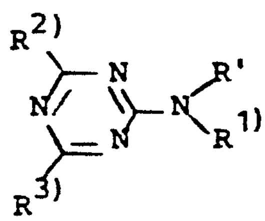

(Ia)

in welcher

R' für Wasserstoff oder Alkenyl steht,

$R^{1)}$ für Alkyl mit mehr als 3 Kohlenstoffatomen,

Alkenyl, Alkinyl, Alkoxyalkyl, Alkoxycarbonylalkyl, Carboxyalkyl und

$R^{2)}$ und $R^{3)}$ gleich oder verschieden sind und für Alkylthio stehen,

dadurch gekennzeichnet, daß man

a) 2-Halogentriazine der Formel (II)

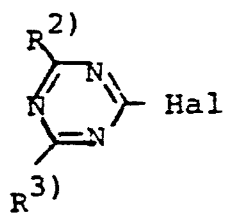

(II)

in welcher

Hal für ein Halogenatom steht und

$R^{2)}$ und $R^{3)}$ die oben angegebene Bedeutung haben,

mit Aminen der Formel (III)

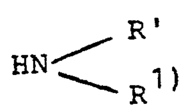

(III)

in welcher

R' und $R^{1)}$ die oben angegebene Bedeutung haben,

gegebenenfalls in Gegenwart einer Lösungs- oder Verdünnungsmittels und gegebenenfalls in Gegenwart eines Säurebindemittels umsetzt oder

b) 2-Aminotriazine der Formel (IV)

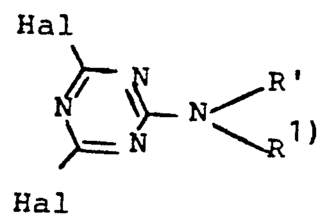

(IV)

in welcher

Hal für Halogen steht und

R' und $R^{1)}$ die weiter oben angegebene Bedeutung haben,

mit Verbindungen der Formel (V) im 1. Schritt

$R^{2)}H$ (V)

und gegebenenfalls mit Verbindungen der Formel (VI) im 2. Schritt

$R^{3)}H$ (VI)

wobei

$R^{2)}$ und $R^{3)}$ die weiter oben angegebene Bedeutung haben,

gegebenenfalls in Gegenwart eines Lösungs- oder Verdünnungsmittels, gegebenenfalls in Gegenwart eines Säurebindemittels umsetzt, oder

c) Verbindungen der Formel (VII)

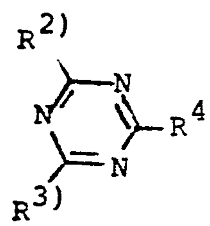

(VII)

worin

$R^4$ für Alkylthio steht und alle Reste $R^{2)}$, $R^{3)}$ und $R^4$ identisch sind,

mit stöchiometrischen Mengen an Aminen der Formel (III) gegebenenfalls in Gegenwart eines Verdünnungs- oder Lösungsmittels bei Temperaturen zwischen 30°C und 200°C umsetzt.